# EUROPÄISCHE PATENTSCHRIFT

(11) **EP 2 250 495 B1**
(45) Veröffentlichungstag und Bekanntmachung des Hinweises auf die Patenterteilung: **09.11.2011**
(21) Anmeldenummer: 09806002.3
(22) Anmeldetag: 20.11.2009
(51) Int. Cl.: B29C 39/00

(54) **VERFAHREN ZUR IDENTIFIZIERUNG VON PRODUKTEN MIT MARKERN BESTEHEND AUS SALZEN VON SELTENERDMETALLEN**
METHOD FOR IDENTIFYING PRODUCTS WITH MARKERS CONSISTING OF SALTS OF RARE EARTH METALS
PROCÉDÉ POUR L'IDENTIFICATION DE PRODUITS, AVEC DES MARQUEURS CONSTITUÉS DE SELS DE MÉTAUX DES TERRES RARES

(30) Priorität: 20.11.2008 DE 102008058177; 16.12.2008 US 201832 P
(43) Veröffentlichungstag der Anmeldung: 17.11.2010
(73) Patentinhaber: EOS GmbH Electro Optical Systems, 82152 Krailling (DE)
(72) Erfinder: KELLER, Peter, 82152 Krailling (DE); MATTES, Thomas, 82205 Gilching (DE); GERSCH, Mandy, 89073 Ulm (DE); OBERHOFER, Johann, 82131 Stockdorf (DE); MAYER, Anton, 82205 Gilching (DE)
(74) Vertreter: Prüfer & Partner GbR European Patent Attorneys
(86) Internationale Anmeldenummer: PCT/EP2009/008272
(87) Internationale Veröffentlichungsnummer: WO 2010/057649

(56) Entgegenhaltungen:
- EP-A1- 1 494 000
- WO-A1-2006/119759
- WO-A2-98/28124
- CN-A- 1 235 936
- JP-A- 2007 113 327
- US-A- 5 677 187
- US-A1- 2003 118 440
- US-B1- 6 974 641

## Beschreibung

Die Erfindung ist auf ein Verfahren gemäss Anspruche 1 gerichtet, das

Bei der schichtweisen Herstellung von Objekten aus pulverförmigen Ausgangsstoffen beispielsweise mittels Lasersintern stellt sich das Problem, dass die pulverförmigen Ausgangsstoffe in ihrem Aussehen nicht zu unterscheiden sind, obwohl es sich um unterschiedliche Pulver handelt. Beispielsweise kann einem Pulver optional ein Flammschutzmittel zugesetzt sein, um eine Feuerbeständigkeit des zu erzeugenden Objektes zu erreichen. Solch ein Zusatz verändert in der Regel nicht das Aussehen des Pulvers. Ob ein Flammschutzmittel zugesetzt wurde, kann dann in der Regel nur durch eine aufwendige Analyse des Pulvers festgestellt werden.

Des Weiteren kann es auch wichtig sein, bei einem bereits hergestellten Objekt bzw. Bauteil festzustellen, aus welchem Pulver es gefertigt wurde. Dies kann z.B. Teil einer Fehleranalyse sein, wenn das Objekt nicht die gewünschten Eigenschaften aufweist. Selbst wenn das Objekt einwandfrei ist, kann es z.B. erwünscht sein, manchmal nach Jahren, den Lieferanten des verwendeten Lasersinterpulvers zu kennen. Hierbei soll bei einer Analyse in der Regel das Objekt nicht beschädigt werden müssen, beispielsweise wenn eine Probe entnommen wird.

WO 2006 / 11 9 759 offenbart die Markierung von Kumstoffen und oxiden Seltener Erden.

Ein Zusatz von Farbpigmenten zu dem Pulver zur Kennzeichnung unterschiedlicher Pulvereigenschaften ist ungeeignet, da dadurch die Endfarbe der zu erzeugenden Objekte beeinflußt wird, was in vielen Fällen unerwünscht ist.

Die Aufgabe der Erfindung ist es daher, ein Verfahren bereitzustellen, dass die Identifizierung von in einem Schichtbauverfahrent hergestellten Bauteilen gestattet, ohne dass das Aussehen des Pulvers und der Bauteile verändert werden.

Die Aufgabe wird gelöst durch ein Verfahren zur Kennzeichnung von in einem Schichtbauverfahren hergestellten Produkten nach Anspruch 1 und eine Verwendung solch eines Pulvers nach Anspruch 8.

Weiterbildungen der Erfindung sind in den Unteransprüchen beschrieben.

Durch das erfindungsgemäße Verfahren ist es insbesondere möglich, gefertigte Bauteile exakt einem bestimmten verwendeten Ausgangspulver zuzuordnen. Damit kann auch noch nach vielen Jahren, wenn Aufzeichnungen verloren gegangen sind oder Lieferketten nicht mehr lückenlos nachverfolgbar sind, festgestellt werden, aus welchem Ausgangsmaterial welchen Herstellers die entsprechenden Teile erzeugt wurden. Insbesondere ist es auch möglich, dass das Pulver nicht nur entsprechend einem bestimmten Hersteller bzw. Herstelldatum gekennzeichnet wird, sondern dass auch ein Hinweis auf denjenigen angebracht wird, der mit diesem Pulver Bauteile erzeugt hat. Durch das erfindungsgemäße Verfahren ist es des weiteren möglich, eine Identifizierung selbst dann vorzunehmen, wenn nur beliebige, kleine Bruchstücke von Bauteilen vorliegen.

Weitere Merkmale und Zweckmäßigkeiten der Erfindung werden im Folgenden anhand eines Ausführungsbeispiels beschrieben.

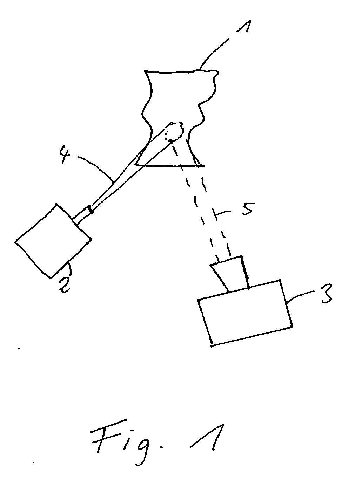

Fig. 1 zeigt einen beispielhaften Aufbau zur Veranschaulichung eines Verfahrens zur Untersuchung eines gekennzeichneten Pulvers oder daraus hergestellten Bauteils.

Zur Kennzeichnung eines in einem Schichtbauverfahren, beispielsweise einem Lasersinterverfahren, als Baumaterial verwendbaren Pulvers dergestalt, dass dessen Eigenschaften oder dessen Herkunft von einem speziellen Hersteller erkennbar sind, wird gemäß der Erfindung das Pulver in einem handelsüblichen Mischer mit einem Markierungspulver vermischt. Damit eine zu starke Änderung der Eigenschaften des Ausgangspulvers durch das Markierungspulver vermieden wird, ist es günstig, wenn der Anteil des Markierungspulvers an der Mischung einen gewissen Prozentsatz, z.B. 20 Gew.-%, nicht überschreitet. Natürlich ist ein noch geringerer Anteil, beispielsweise 10 Gew.-%, oder besser noch zwischen 0.1 und 10 Gew.-%, noch vorteilhafter. Allerdings sinkt mit dem Anteil des Markierungspulvers auch die Wahrscheinlichkeit, noch in kleinen Proben der Gesamtmischung Markierstoffpartikel zu finden. Im Hinblick auf eine Pulveridentifizierung auch bei kleinen Pulvermengen ist es auch wichtig, dass die Vermischung so vollständig stattfindet, dass das Endprodukt möglichst homogen ist.

Da die Eigenschaften der herzustellenden Objekte durch die Hinzufügung des als Identifikationsmittel dienenden Markierungsstoffes nicht verändert werden sollen, muß der Markierungsstoff farblos sein, oder aber in so einem geringen Mengenverhältnis hinzugefügt sein, dass keine Veränderung der Farbe des Ausgangspulvers erkennbar ist. Um dennoch ein Pulver oder Bauteil identifizieren zu können, wird erfindungsgemäß als Markierungsstoff eine Substanz gewählt, die eine Lumineszenz zeigt, wenn sie mit Licht einer Wellenlänge außerhalb des sichtbaren Bereichs, beispielsweise IR-Licht oder UV-Licht bestrahlt wird. Für die Identifizierung muß dann das von dem lumineszierenden Stoff emittierte Licht auf seine Wellenlänge und/oder Intensität hin untersucht werden. Dadurch kann allein anhand des Vorliegens einer Lumineszenzemission bestimmt werden, ob ein Markierungsstoff dem Pulver beigesetzt wurde. Falls eine Lumineszenzemission bei Bestrahlung festzustellen ist, kann anhand der eingestrahlten (anregenden) und/oder der emittierten Wellenlänge(n) ermittelt werden, welcher Markierungsstoff zugesetzt wurde.

Ein Pulver kann nun dadurch gekennzeichnet werden, dass ein Markierungsstoff zugesetzt wird, welcher eine ganz bestimmte Wellenlänge oder mehrere charakteristische Wellenlängen oder einen bestimmten Wellenlängenbereich emittiert. Natürlich ist es auch möglich, einen Markierungsstoff zu verwenden, der eine Lumineszenz in verschiedenen Wellenlängenbereichen zeigt. Allgemein ist eine Identifizierung über die Registrierung einer ganz bestimmten Spektralverteilung im emittierten Licht möglich.

Das gekennzeichnete Pulver kann als Baumaterial in einem beliebigen Schichtbauverfahren zum Herstellen von dreidimensionalen Objekten verwendet werden, also z.B. in einem selektiven Lasersinter- oder Laserschmelzverfahren, bzw. einem selektiven Elektronenstrahl- oder Infrarot- -sinter- oder -schmelzverfahren oder aber einem 3D-Druckverfahren, bei dem zum Verfestigen des Materials ein Bindemittel aufgespritzt wird. Die genannten Verfahren sind unter anderem in WO 90/03893 und US 6,375,874 B1 beschrieben.

Ein beispielhafter Aufbau zur Untersuchung eines mittels markiertem Pulver hergestellten Bauteils ist in Fig. 1 dargestellt. Auf ein lasergesintertes Bauteil 1 wird dabei mit Hilfe einer UV-Lichtquelle 2 UV-Licht 4 gestrahlt. Ein Teil des durch die Bestrahlung angeregten Lumineszenzlichtes 5 wird durch einen Detektor 3 erfasst.

Alternativ zu UV-Licht kann auch Licht mit einer anderen Wellenlänge außerhalb des sichtbaren Bereichs, z.B. im IR-Bereich, in einer bevorzugten Ausbildung der Erfindung im nahen Infrarot (NIR), noch bevorzugter zwischen 900 nm und 1000 nm, für die Anregung der Lumineszenz verwendet werden. Des Weiteren ist auch eine Anregung der Lumineszenz mittels ionisierender Strahlung (Teilchenstrahlung oder Röntgenstrahlung) möglich.

Der Detektor für die Untersuchung des Lumineszenzlichts kann dabei eine einfache Photodiode oder aber auch ein CCD oder Pixelsensor sein, der die Lichtmenge erfaßt. Der Nachweis der Anwesenheit eines Markierstoffes geschieht im einfachsten Fall durch Vergleich der Lichtemissionen des Pulvers oder fertig gestellten Bauteils mit und ohne Anregungslicht. Die Identifizierung der Wellenlängen des Lumineszenzlichts kann z.B. durch Filtervorsätze vor dem Detektor realisiert werden, wobei die Filtervorsätze jeweils nur eine Transmission in einem begrenzten Wellenlängenbereich zeigen. Denkbar ist aber auch die Verwendung anderer Aufbauten, die eine Spektralzerlegung liefern (z.B. Prismen, Gitter, etc.). Die Spektralzerlegung kann dabei auch im Detektor selbst stattfinden.

Zur Bestimmung der Konzentration an Markierungsstoffen im Pulver bzw. Bauteil wird beispielsweise die emittierte Menge an Lumineszenzlicht einmal ohne vorgesetztes Filter und zum anderen mit einem vorgesetzten Filter gemessen, wobei das Filter nur im Bereich des Lumineszenzspektrums eine Transmission zeigt. Auf diese Weise kann die Menge des Lumineszenzlichts in Relation zu der insgesamt vom Pulver oder Bauteil reflektierten Lichtmenge, die auf den Detektor fällt, gesetzt werden. Unter der Voraussetzung einer geeigneten Kalibration des Systems kann man dann die Menge des zugesetzten Markierstoffs bestimmen, falls es die zugesetzte Markierstoffmenge ist, die zur Kodierung von Information verwendet wird.

In einer abgewandelten Ausführungsform werden dem Pulver zwei verschiedene Markierungsstoffe zugesetzt, die bei der Lumineszenz eine Lichtemission in verschiedenen Wellenlängenbereichen zeigen und/oder verschiedene anregende Wellenlängen besitzen. Eine bestimmte Codierung kann dann erzeugt werden durch Bemessen des Mengenverhältnisses der beiden zugesetzten Markiersubstanzen zueinander. Das Mengenverhältnis wird dann bei der Untersuchung des Pulvers oder Bauteils dadurch ermittelt, dass die in den beiden unterschiedlichen Wellenlängenbereichen emittierten Lichtmengen zueinander in Relation gesetzt werden. Auf diese Weise kann eine entsprechende Codierung gelesen werden. Natürlich können auch mehr als zwei unterschiedliche Markiersubstanzen zugesetzt werden. Des Weiteren ist es auch möglich, einen Markierungsstoff zu verwenden, der eine Lumineszenz in verschiedenen Wellenlängenbereichen zeigt.

Selbst wenn die Emissionsbereiche zweier Markiersubstanzen einander überlappen, können bei Verwendung eines Spektrometers zur Analyse der Lumineszenzstrahlung dennoch die relativen Mengenverhältnisse der beiden Substanzen ermittelt werden.

Das beschriebene Verfahren läßt sich auf alle möglichen Pulver anwenden, also insbesondere auf Polymerpulver, Metallpulver und Sandsinterpulver. Da für einige der genannten Pulver sehr hohe Temperaturen während beispielsweise eines Sinter- oder Schmelzprozesses auftreten, ist eine wichtige Anforderung an die Auswahl der Markierstoffe die, dass die Markiersubstanzen durch die hohen Temperaturen, die während des Bauvorgangs auftreten, nicht beeinträchtigt werden. Es wurde gefunden, dass bei Verwendung von Salzen der seltenen Erden eine Temperaturbeständigkeit für die normalerweise bei Lasersinterverfahren auftretenden Temperaturen gegeben ist. Hierzu zählen z.B. Oxide der seltenen Erden oder Oxi-Sulfide oder auch Fluoride, die mit kleinen Beimengungen anderer Elemente, die ebenfalls der Gruppe de seltenen Erden entstammen, dotiert sind, um die gewünschte Lumineszenz zu erzeugen.

Die Markiersubstanz kann in besonders vorteilhafter Weise so zugemischt werden, dass die Teilchen der Markiersubstanz auf der Oberfläche der Pulverteilchen eingebettet werden. Auf diese Weise kann man jedes einzelne Pulverteilchen markieren. Hierzu werden die Markierungssubstanz und die Pulverteilchen beispielsweise einem in der EP 0 555 947 A1 beschriebenen Verfahren für die Oberflächenbehandlung von Teilchen unterzogen. Dabei werden die Partikel einer von mehreren, untereinander verbundenen Aufschlagkammern zugeführt, die mit einer Drehscheibe mit Aufschlagstiften versehen sind, sowie mit einem Aufprallring, so dass das Gemisch einem Schlagprallvorgang ausgesetzt wird, wodurch ein durch diesen Vorgang erzeugter Luftstrom von dem Pulvergemisch getrennt und kontinuierlich aus der Aufschlagkammer freigegeben wird, wobei der Aufschlagvorgang wiederholt wird und das Pulvergemisch vorübergehend in der Aufschlagkammer ruhen kann, bevor es in die darauffolgende Kammer weitergeleitet wird. Versuche mit einer solchen handelsüblichen Pulverbehandlungsmaschine NHS-1 der Firma Nara ergaben, dass die Behandlungsdauer bei einer Umdrehungszahl von 8.000 U/min mindestens eine Minute betragen muß (bei Raumtemperatur).

Es soll abschließend noch betont werden, dass man unterschiedliche Markiersubstanzen nicht nur daran erkennen kann, welches Licht nach einer Anregung emittiert wird, sondern auch daran, durch welche Wellenlänge(n) die Lumineszenz angeregt wird.

## Patentansprüche

1. Verfahren zur Kennzeichnung von in einem Schichtbauverfahren hergestellten Produkten mit den folgenden Schritten:
Vermischen des als Baumaterial zu verwendenden Pulvers mit mindestens einem Salz eines Metalls der seltenen Erden, wobei das Salz die Eigenschaft aufweist, dass es eine Lumineszenz zeigt, wenn es mit Photonen einer Wellenlänge außerhalb des sichtbaren Spektrums oder mit Teilchenstrahlung bestrahlt wird und
Verwenden des vermischten Pulvers als Baumaterial in einem Verfahren zur Herstellung eines dreidimensionalen Objektes durch schichtweises Aufbringen und Verfestigen des Pulvers an den dem Querschnitt des herzustellenden Objektes in der jeweiligen Schicht entsprechenden Stellen, wobei die Verfestigung durch Einwirkung eines Lasers oder einer anderen Energiequelle auf das Pulvermaterial oder durch selektives Aufbringen eines Bindemittels geschieht.

2. Verfahren nach Anspruch 1, bei dem mehrere unterschiedliche Salze der seltenen Erden zugefügt werden, deren spektrale Emissionen bei der Lumineszenz zueinander unterschiedlich sind.

3. Verfahren nach Anspruch 1, bei dem in Abhängigkeit von der Identität des Pulverherstellers, der Identität des Anwenders oder in Abhängigkeit vom Herstellungsort des mittels des Schichtbauverfahrens hergestellten Bauteils oder in Abhängigkeit von der Pulverzusammensetzung das als Markierung zuzusetzende Salz bzw. die als Markierung zuzusetzenden Salze ausgewählt werden.

4. Verfahren nach einem der Ansprüche 1 bis 3, bei dem die Salze durch scherendes Mischen mit dem Pulver vermischt werden.

5. Verfahren nach einem der Ansprüche 1 bis 3, bei dem das oder die Salze mit dem Pulver dergestalt vermischt werden, dass die Pulverpartikel einem Schlagprallvorgang ausgesetzt werden, der dazu führt, dass das Salz auf der Oberfläche der Pulverteilchen eingebettet wird.

6. Verfahren nach einem der Ansprüche 1 bis 5, bei dem eines oder mehrere der folgenden Salze verwendet werden: dotierte Oxide, Oxi-Sulfide oder auch Fluoride der seltenen Erden.

7. Verfahren nach Anspruch 6, bei dem Yttriumoxid Y₂O₃, Yttriumoxisulfid Y₂O₂S oder Natrium-Yttrium-Flourid NaYF₄, jeweils dotiert mit Erbium oder einem anderen Element der seltenen Erden, verwendet werden.

8. Verwendung eines der Verfahren der vorangehenden Ansprüche zur Codierung von Informationen bezüglich der Pulverzusammensetzung, bezüglich des Pulverherstellers oder - verarbeiters oder des Herstellungsortes des Pulvers.

9. Verwendung nach Anspruch 8, bei der die Codierung durch Wahl der Menge des zugesetzten Salzes geschieht.

10. Verwendung nach Anspruch 8, bei der die Codierung durch Auswahl eines bestimmten Salzes oder mehrerer bestimmter Salze, die zugefügt werden, geschieht.

11. Verwendung nach Anspruch 8, bei der die Codierung durch die Auswahl einer bestimmten Kombination von unterschiedlichen zugesetzten Salzen geschieht.

12. Verwendung nach Anspruch 8, bei der die Codierung durch die Wahl einer bestimmten Mengenrelation zwischen den mindestens zwei zugefügten Salzen geschieht.

13. Verfahren nach einem der Ansprüche 1 bis 7, bei dem es sich bei dem Verfahren zur Herstellung eines dreidimensionalen Objektes um ein selektives Lasersinterverfahren oder ein selektives Laserschmelzverfahren handelt.

## Claims

1. Method for labelling powders that can be used as building material in a layer-additive manufacturing method having the following steps:
mixing the powder with at least one salt of a metal of the rare earths, wherein the salt has the property that it shows a luminescence when being irradiated with photons having a wavelength outside of the visible spectrum or with particle radiation, and
using the mixed powder as building material in a method for manufacturing a three-dimensional object by applying and solidifying the powder layer-wise at those positions in the respective layer that correspond to the cross-section of the object to be manufactured, wherein the solidification is effected by the action of a laser or another energy source onto the powder material or is effected by selectively applying a binder.

2. Method according to claim 1, in which a plurality of different salts of the rare earths is added, the spectral emissions of which during the luminescence are different from one another.

3. Method according to claim 1, in which the salt that is to be added as marker or the salts that are to be added as markers is/ are selected depending on the identity of the powder producer, the identity of the applicant or depending on the place of manufacture of the part that has been manufactured by means of the layer-additive manufacturing method or depending on the powder composition.

4. Method according to one of claims 1 to 3, in which the salts are mixed with the powder by shear mixing.

5. Method according to one of claims 1 to 3, in which the salt(s) are mixed with the powder such that the powder particles are subjected to an impact striking action leading to an embedding of the salt on the surface of the powder particles.

6. Method according to one of claims 1 to 5, in which one or more of the following salts are used: doped oxides, oxysulfides or also fluorides of the rare earths.

7. Method according to claim 6, in which yttrium oxide Y₂O₃, yttrium oxysulfide Y₂O₂S or sodium yttrium fluoride NaYF₄, in each case doped with erbium or another element of the rare earths, are used.

8. Use of one of the methods of the preceding claims for encoding information related to the powder composition, related to the powder producer or powder user or related to the place of manufacture of the powder.

9. Use according to claim 8, in which the encoding is effected by selecting the amount of the added salt.

10. Use according to claim 8, in which the encoding is effected by selecting a specific salt or several specific salts that are added.

11. Use according to claim 8, in which the encoding is effected by selecting a specific combination of differing added salts.

12. Use according to claim 8, in which the encoding is effected by selecting a specific relation between the amounts of the at least two added salts.

13. Method according to one of claims 1 to 7, in which the method for manufacturing a three-dimensional object is a selective laser sintering method or a selective laser melting method.

## Revendications

1. Procédé de marquage de produits fabriqués dans un procédé de construction par couches présentant les étapes suivantes :
mélange de la poudre à utiliser comme matériau de construction avec au moins un sel d'un métal des terres rares, le sel présentant la propriété qu'il présente une luminescence lorsqu'il est irradié avec des photons d'une longueur d'onde en dehors du spectre visible ou avec un rayonnement de particules et
utilisation de la poudre mélangée comme matériau de construction dans un procédé de fabrication d'un objet en trois dimensions par application couche par couche et consolidation de la poudre aux emplacements correspondants à la section de l'objet à fabriquer dans la couche respective, la consolidation s'effectuant en faisant agir un laser ou une autre source d'énergie sur le matériau en poudre ou par application sélective d'un liant.

2. Procédé selon la revendication 1, dans lequel plusieurs sels différents des terres rares sont ajoutés, dont les émissions spectrales sont différentes les unes des autres en ce qui concerne la luminescence.

3. Procédé selon la revendication 1, dans lequel le sel ou les sels à ajouter comme marquage sont sélectionnés en fonction de l'identité du fabricant de poudre, de l'identité de l'utilisateur ou en fonction du lieu de fabrication de l'élément de construction fabriqué au moyen du procédé de construction par couches ou en fonction de la composition de la poudre.

4. Procédé selon l'une des revendications 1 à 3, dans lequel les sels sont mélangés avec la poudre par mélange cisaillant.

5. Procédé selon l'une des revendications 1 à 3, dans lequel le ou les sels sont mélangés avec la poudre, de telle sorte que les particules de poudre sont exposées à un processus de percussion, qui aboutit à ce que le sel est noyé sur la surface des particules de poudre.

6. Procédé selon l'une des revendications 1 à 5, dans lequel un ou plusieurs des sels suivants sont utilisés : oxydes dopés, oxy-sulfures ou également fluorures des terres rares.

7. Procédé selon la revendication 6, dans lequel de l'oxyde d'yttrium Y₂O₃, de l'oxysulfure d'yttrium Y₂O₂S ou du fluorure de sodium-yttrium NaYF₄, dopés respectivement avec de l'erbium ou un autre élément des terres rares, sont utilisés.

8. Utilisation de l'un des procédés des revendications précédentes pour le codage d'informations concernant la composition de la poudre, concernant le fabricant ou le transformateur de la poudre ou le lieu de fabrication de la poudre.

9. Utilisation selon la revendication 8, lors de laquelle le codage s'effectue en choisissant la quantité du sel ajouté.

10. Utilisation selon la revendication 8, lors de laquelle le codage s'effectue par sélection d'un sel défini ou de plusieurs sels définis qui sont ajoutés.

11. Utilisation selon la revendication 8, lors de laquelle le codage s'effectue par la sélection d'une combinaison déterminée de différents sels ajoutés.

12. Utilisation selon la revendication 8, lors de laquelle le codage s'effectue par le choix d'une relation de quantités définie entre les au moins deux sels ajoutés.

13. Procédé selon l'une des revendications 1 à 7, dans lequel, en ce qui concerne le procédé de fabrication d'un objet en trois dimensions, il s'agit d'un procédé sélectif de frittage au laser ou d'un procédé sélectif de fusion au laser.
